# EUROPEAN PATENT SPECIFICATION

(11) **EP 2 758 087 B1**
(45) Date of publication and mention of the grant of the patent: **04.07.2018**
(21) Application number: 12842642.6
(22) Date of filing: 22.10.2012
(51) Int. Cl.: A61L 27/26, A61L 27/50

(54) **COLLAGEN-POLYSACCHARIDE MATERIALS MIMICKING BLOOD VESSELS, TISSUES AND BONES**
BLUTGEFÄSSEN, GEWEBE UND KNOCHEN IMITIERENDE KOLLAGEN-POLYSACCHARID-MATERIALIEN
MATÉRIAUX À BASE DE COLLAGÈNE-POLYSACCHARIDE IMITANT LES VAISSEAUX SANGUINS, LES TISSUS ET LES OS

(30) Priority: 21.10.2011 US 201161550104 P
(43) Date of publication of application: 30.07.2014
(73) Proprietor: Nitta Casings Inc., Bridgewater, NJ 08807 (US)
(72) Inventor: BATTERSBY, Richard, E., Frenchtown, NJ 08825 (US); GOLDFARB, Eugene, Marlboro, NJ 07746 (US); MATHEWS, David, E., Somerville, NJ 08876 (US); SHEDLOCK, Michael, T., Easton, PA 18045 (US); GUZMAN, Norberto, A., East Brunswick, NJ 08816 (US)
(74) Representative: Patentanwälte Bauer Vorberg Kayser
(86) International application number: PCT/US2012/061299
(87) International publication number: WO 2013/059780

(56) References cited:
- CN-A- 101 845 226
- US-A- 3 782 977
- US-A- 4 182 054
- US-A- 4 182 054
- US-A1- 2001 014 662
- US-A1- 2005 031 741
- US-A1- 2005 031 741
- US-A1- 2008 107 744
- US-A1- 2009 005 881
- US-A1- 2009 022 775
- US-B1- 6 235 328

## Description

### Field of the Invention

The present invention also relates to a process for testing phlebotomical, surgical or orthopedic instrumentation and/or practicing phlebotomical, surgical or orthopedic procedures using a medical device comprising a collagen-polysaccharide material which may be in the form of a hollow tube that mimics a blood vessel, a sheet that mimics a tissue, or a solid that mimics a bone.

### Discussion of the Background

Collagen is a major fibrous protein constituent of cartilage, skin, bones, tendons, and other connective tissues of animals, as well as of skin, bones and scales of aquatic animals.

Collagen is present in all types of multicellular organisms and is probably the most abundant animal protein in nature. Collagen is located in the extracellular matrix of connective tissues and contributes to tissue integrity and mechanical properties.

Collagen molecules are composed of three polypeptide chains called alpha chains. The alpha chains contain about 1000 amino acid residues and, with the exception of short sequences at the ends of the chains, every third amino acid in each chain is glycine. The molecular formula of an alpha chain can thus be approximated as (X-Y-Gly)₃₃₃, where X and Y represent amino acids other than glycine. In collagen from mammals and birds about 100 of the X positions are proline, and about 100 of the Y positions are hydroxyproline. Collagen molecules have been classified into at least 29 types in the order in which they were purified and characterized, and grouped into classes according to their physicochemical properties.

Due to their excellent biocompatibility, biodegradability, and ease of extraction, purification and processing, collagen molecules have found use as a versatile biomaterial in numerous medical and pharmaceutical applications. Furthermore, collagen is used in the food and beverage industry, cosmetic industry, and nutraceutical industry in a variety of compositions.

US 6 235 328 B1 discloses a process and apparatus for the production of co-extruded collagen coated foodstuffs, wherein the collagen gel consists essentially of between about 4% and about 10% collagen, between about 0.1% and about 2% cellulose, between about 0.05% and about 0.5% hydrochloric acid, and water.

CN 101 845 226 A discloses a dialdehyde carboxymethyl cellulose-collagen frozen gel and a preparation method thereof. The method comprises the steps of uniformly mixing 1 to 3 mass percent of the aqueous solution of collagen and 0.01 to 1 mass percent of the aqueous solution of dialdehyde carboxymethyl cellulose, then injecting the mixed solution into a mold and storing the mixed solution in a low-temperature reactor at the temperature of -40 to 0 DEG C for 1 to 7 days; and finally, taking the obtained product out and slowly unfreezing the obtained product to obtain the dialdehyde carboxymethyl cellulose-collagen frozen gel. The dialdehyde carboxymethyl cellulose-collagen freezing gel prepared by the method is improved in mechanical property, thermal stability and enzymatic degradation resistance at the same time of maintaining the biological activity of collagen gel per se, has the advantages of porosity, hydrophilicity, water absorbing and preserving property, good market application prospect and the like, and is widely used in biomedical fields, such as biologic scaffolds, cell culture, drug delivery, tissue engineering, wound and burning treatment and the like.

US 4 182 054 A discloses an apparatus to artificially simulate arteries of a limb for training medical personnel to puncture arteries and veins. The device comprises a hollow arm shaped member constructed of a resilient material having two grooves formed on the exterior thereof in the area of the elbow and wrist simulating the brachial artery and radial artery area. A resilient artery is formed of tubing which is positioned through the hollow bore of the arm and through the grooves in the elbow and wrist area. A latex skin is positioned over the arm to cover the tubing and grooves to simulate the skin. The tubing is attached to a liquid container above the arm to simulate blood and cause a arterial or venous pressure for a lifelike back flow of liquid into the syringe.

Unembalmed anatomical materials simulating a "real world" tissue for testing phlebotomical, surgical or orthopedic instrumentation during research and development and/or for practicing phlebotomical, surgical or orthopedic procedures during the training of healthcare professionals (e.g., physicians, phlebotomists, surgeons, and medical students) suffer from a number of disadvantages including but not limited to a short shelf-life, rapid decomposition and the emission of foul odors, as well as the reliance on obtaining the unembalmed anatomical materials from animals and human cadavers.

Accordingly, there remains a critical need for a medical device in the form of an artificial blood vessel, an artificial tissue, and/or an artificial bone that is manufactured to replicate a naturally occurring blood vessel, tissue, and bone for testing phlebotomical, surgical or orthopedic instrumentation during research and development and/or for practicing phlebotomical, surgical or orthopedic procedures during the training of healthcare professionals (e.g., physicians, phlebotomists, surgeons, and medical students) while obviating the above-identified problems.

### SUMMARY OF THE INVENTION

The present invention provides a process for testing phlebotomical, surgical or orthopedic instrumentation and/or practicing phlebotomical, surgical or orthopedic procedures using a medical device comprising a collagen-cellulose material comprising 1.0-9.0 wt. % of a collagen, based on a total weight of the collagen-cellulose material; 0.2-3.0 wt. % of cellulose or a derivative thereof, based on the total weight of the collagen-cellulose material; 0.5-6.5 wt. % of at least one acid selected from an inorganic acid, an organic acid, and mixtures thereof, based on the total weight of the collagen-cellulose material; and water; and wherein the collagen-cellulose material is in the form of an artificial blood vessel, an artificial tissue, and/or an artificial bone.

In an exemplary aspect of the present invention the collagen-cellulose material comprising: 3.0-7.0 wt. % of a collagen, based on a total weight of the collagen-cellulose material; 0.8-2.0 wt. % of cellulose or a derivative thereof, based on the total weight of the collagen-cellulose material; 1.0-6.0 wt. % of at least one acid selected from an inorganic acid, an organic acid, and mixtures thereof, based on the total weight of the collagen-cellulose material; and water.

In a further exemplary aspect of the present invention the collagen-cellulose material comprising: 3.0-7.0 wt. % of a collagen, based on a total weight of the collagen-cellulose material; 0.8-2.0 wt. % of cellulose or a derivative thereof, based on the total weight of the collagen-cellulose material; 1.0-6.0 wt. % of at least one acid selected from an inorganic acid, an organic acid, and mixtures thereof, based on the total weight of the collagen-cellulose material; 0.1-25 wt. % of one or more compounds selected from pectin, sodium citrate, ammonium sulfate, citric acid, a crosslinking agent (e.g., glutaraldehyde and/or hydroxyacetaldehyde), an aluminum salt, a sugar, a sugar derivative obtained from a caramelization process, a sugar mixture comprising a sugar or a derivative thereof and a crosslinking agent (e.g., MAILLOSE®), a browning agent, glycerin, an antimicrobial agent (e.g., sodium benzoate and/or hypochlorite), mineral oil, poly(lactic-co-glycolic acid) (PLGA), hydroxyapatite, a surfactant, a plasticizer, a dye, and mixtures thereof, based on the total weight of the collagen-cellulose material; and water.

The artificial blood vessel (e.g., an artery, a vein, or a capillary) may have any desired dimension, including any desired length, aspect ratio, inner diameter, and/or outer diameter. The artificial tissue (e.g., a slab, a pad, a membrane, or a film) may have any desired dimension, including any desired length, width, and/or thickness. The artificial bone may have any desired dimension, including any desired shape, length, width, thickness, and/or outer diameter. The artificial blood vessel, the artificial tissue, and the artificial bone may also have a desired tensile strength, modulus of elasticity, flexibility, porosity, compressibility, decompressibility, hardness, degree of crosslinking, wetness, sealability, and/or stability.

The foregoing discussion exemplifies certain aspects of the present invention. Additional exemplary aspects of the present invention are discussed in the following detailed description of the invention.

### DETAILED DESCRIPTION OF THE INVENTION

Unless specifically defined, all technical and scientific terms used herein have the same meaning as commonly understood by a skilled artisan in the relevant technological field.

The present invention provides a method for testing phlebotomical, surgical or orthopedic instrumentation and/or practicing a phlebotomical, surgical or orthopedic procedure comprising performing testing of the phlebotomical, surgical or orthopedic instrumentation or practicing of the phlebotomical, surgical or orthopedic procedure on a medical device comprising a collagen-cellulose material comprising: 1.0-9.0 wt. % of a collagen, based on a total weight of the collagen-cellulose material; 0.2-3.0 wt. % of cellulose or a derivative thereof, based on the total weight of the collagen-cellulose material; 0.5-6.5 wt. % of at least one acid selected from an inorganic acid, an organic acid, and mixtures thereof, based on the total weight of the collagen-cellulose material; and water; and wherein the collagen-cellulose material is in a form of an artificial blood vessel, an artificial tissue, and/or an artificial bone.

The medical device comprising the collagen-cellulose material is in the form of an artificial blood vessel (e.g., an artificial artery, an artificial vein, or an artificial capillary), an artificial bone (e.g., femur bone, tooth), or an artificial tissue including an artificial connective tissue (e.g., an artificial tendon, an artificial ligament, or an artificial organ), an artificial epidermal tissue (e.g., artificial skin), an artificial muscle tissue, or an artificial nerve tissue.

The artificial blood vessel, the artificial tissue, and the artificial bone may also have a desired tensile strength, modulus of elasticity, flexibility, porosity, compressibility, decompressibility, hardness, degree of crosslinking, wetness, sealability, and/or stability.

The artificial blood vessel is in a form of a hollow tube that may have any desired dimension, including any desired length, inner diameter, and/or outer diameter. Although the hollow tube generally has a circular cross-sectional shape, the hollow tube may have any desired cross-sectional shape including for example irregular, elliptical, triangular, square, pentagonal, hexagonal, etc. The hollow tube may be in the form of a bundle of a plurality of hollow tubes. The hollow tube may have any desired length including for example from 1.0 cm to 100 m. The hollow tube may have any desired internal and/or external diameter including for example from 0.10 mm to 50.0 mm.

The artificial tissue is in a form of a sheet that may have any desired dimension, including any desired length, width and/or thickness. The sheet may have a rectangular cross-sectional shape of any desired dimension. The sheet may have any desired length, width and/or thickness including for example from 0.10 mm to 100 m.

The artificial bone is in a form of a solid that may have any desired dimension, including any desired shape, length, width, thickness, and/or outer diameter.

The medical device may further comprise a synthetic polymer, a natural polymer, an elastomer, a plastic, a thermoplastic, a synthetic rubber, a natural rubber, a silicone rubber, a hydrogel, a ceramic, a metal, an adhesive, a cement, wood, a dye, a pigment, an actual human blood vessel, bone or tissue, an actual animal blood vessel, bone or tissue, and combinations thereof.

The medical device may further comprise a support member for supporting the artificial blood vessel, the artificial bone, and/or the artificial tissue. Non-limiting examples of the support member include a mannequin arm or an artificial arm for supporting the artificial blood vessel, a stand for supporting the artificial bone, and/or a tray for supporting the artificial tissue.

In an exemplary aspect of the present invention the medical device comprising the collagen-cellulose material in the form of an artificial blood vessel, and a support member that may be in the form of a mannequin arm or an artificial arm for supporting the artificial blood vessel, which is useful for practicing phlebotomical procedures during the training of healthcare professionals (e.g., physicians, phlebotomists, surgeons, and medical students).

The medical device comprising the collagen-cellulose material has a number of advantages including obviating the need to test or practice phlebotomical, surgical or orthopedic instrumentation and procedures on animals and human cadavers.

The present invention provides a collagen-cellulose material comprising 1.0-9.0 wt. % of a collagen, including for example 1.5-8.5 wt. %, 2.0-8.0 wt. %, 2.5-7.5 wt. %, 3.0-7.0 wt. %, 3.5-6.5 wt. %, 4.0-6.0 wt. %, 4.5-5.5 wt. %, or 5.0 wt. %, based on a total weight of the collagen-cellulose material. The collagen is preferably present in an amount of 4.0-6.0 wt. %, 3.5-5.5 wt. %, or 3.0-5.0 wt. %, based on a total weight of the collagen-cellulose material. The collagen is particularly preferably present in an amount of 3.5-5.5 wt. %, based on a total weight of the collagen-cellulose material.

The present invention provides a collagen-cellulose material comprising 0.2-3.0 wt. % of cellulose or a derivative thereof, including for example 0.4-2.8 wt. %, 0.6-2.6 wt. %, or 0.8-2.4 wt. %, 1.0-2.2 wt. %, 1.2-2.0 wt. %, 1.4-1.8 wt. %, or 1.6 wt. %, based on a total weight of the collagen-cellulose material. The cellulose or the derivative thereof is preferably present in an amount of 0.6-2.2 wt. %, 0.8-2.0 wt. %, 1.0-1.8 wt. %, 1.2-1.6 wt. %, or 1.4 wt. %, based on a total weight of the collagen-cellulose material. The cellulose or the derivative thereof is particularly preferably present in an amount of 0.8-2.0 wt. %, based on a total weight of the collagen-cellulose material.

The cellulose may be obtained/derived from any plant source or material (e.g., wood). A particularly preferred source of cellulose is pectin-rich plant materials including for example peel (especially albedo, the white portion) of citrus fruits (especially lemon, lime, orange, and grapefruit), apple pomace, sugar beet pulp, sunflower heads, carrots, potatoes, tomatoes, and combinations thereof. For example, lemon peel contains approximately 2-4 wt. % of pectin when fresh and 20-40 wt. % of pectin when dried, whereas dried apple pomace contains approximately 10-20 wt. % of pectin. Cellulose obtained/derived from wood and/or orange peel is particularly preferred. Cellulose obtained/derived from orange peel is especially preferred because of a lower viscosity and a higher pectin content relative to that of wood.

The cellulose may exist in one or more various forms including for example powdered cellulose, microcrystalline cellulose, cellulose fibers, microfibrillated cellulose, and mixtures thereof. The microfibrillated cellulose is a nanocellulose material composed of nanosized cellulose fibrils having a high aspect ratio with a typical width of 5-20 nm and a typical length of up to 2,000 nm.

The derivative of cellulose may be an ether derivative of cellulose, non-limiting examples of which include methylcellulose, ethylcellulose, ethylmethylcellulose, hydroxyethylcellulose, hydroxyethylmethylcellulose, hydroxypropylcellulose, hydroxypropylmethylcellulose, ethylhydroxyethylcellulose, carboxymethylcellulose, carboxyethylcellulose, and mixtures thereof.

The derivative of cellulose may be an ester derivative of cellulose, non-limiting examples of which include cellulose acetate, cellulose triacetate, cellulose propionate, cellulose acetate propionate, cellulose acetate butyrate, cellulose nitrate, cellulose sulfate, and mixtures thereof.

The cellulose component of the collagen-cellulose material of the present invention may be partially substituted with, or completely replaced by, one or more polysaccharides other than cellulose. Non-limiting examples of the one or more polysaccharides other than cellulose include alginate, α-glucan, amylopectin, amylose, arabinoxylan, β-glucan, carrageenan, chitin, chitosan, cyclodextrin, dextran, dextrin, fructan, gellan gum, glucan, glycogen, hemicellulose, inulin, maltodextrin, pectin, starch, and xanthan gum. Preferred collagen-polysaccharide materials of the present invention include a collagen-cellulose material, a collagen-alginate material, and mixtures thereof. A collagen-cellulose material represents a particularly preferred collagen-polysaccharide material of the present invention.

The present invention provides a collagen-cellulose material comprising 0.5-6.5 wt. % of at least one acid selected from an inorganic acid, an organic acid, and mixtures thereof, including for example 1.0-6.0 wt. %, 1.5-5.5 wt. %, 2.0-5.0 wt. %, 2.5-4.5 wt. %, 3.0-4.0 wt. %, or 3.5 wt. %, based on a total weight of the collagen-cellulose material. The at least one acid is preferably present in an amount of 1.0-6.0 wt. %, 1.5-5.5 wt. %, 2.0-5.0 wt. %, 2.5-4.5 wt. %, or 3.0-4.0 wt. %, based on a total weight of the collagen-cellulose material. The at least one acid is particularly preferably present in an amount of 2.0-5.0 wt. %, 2.5-4.5 wt. %, or 3.0-4.0 wt. %, based on a total weight of the collagen-cellulose material.

The acid may be one or more inorganic acids. Non-limiting examples of the inorganic acid include hydrochloric acid, nitric acid, phosphoric acid, sulfuric acid, boric acid, hydrofluoric acid, hydrobromic acid, perchloric acid, and mixtures thereof. Preferred inorganic acids include hydrochloric acid, phosphoric acid, sulfuric acid, and mixtures thereof. A particularly preferred inorganic acid is hydrochloric acid.

The acid may be one or more organic acids. Non-limiting examples of the organic acid include formic acid, acetic acid, propionic acid, butyric acid, valeric acid, caproic acid, and mixtures thereof. Preferred organic acids include formic acid, acetic acid, propionic acid, and mixtures thereof. A particularly preferred organic acid is acetic acid.

The acid may be a mixture of one or more inorganic acids and one or more organic acids. A weight ratio of the one or more inorganic acids to the one or more organic acids includes for example 10/1 to 1/10, 9/1 to 1/9, 8/1 to 1/8, 7/1 to 1/7, 6/1 to 1/6, 5/1 to 1/5, 4/1 to 1/4, 3/1 to 1/3, 2/1 to 1/2, and 1/1. An inorganic acid to organic acid weight ratio of 9/2 to 2/9, 8/3 to 3/8, 7/4 to 4/7, 6/5 to 5/6, and 1/1 is preferred. An inorganic acid to organic acid weight ratio of 1/1 is particularly preferred. The acid is preferably a mixture of hydrochloric acid and acetic acid in a weight ratio of 3/1 to 1/3, 2/1 to 1/2, or 1/1.

The present invention provides preferably a collagen-cellulose material comprising: 3.0-7.0 wt. % of a collagen, based on a total weight of the collagen-cellulose material; 0.8-2.0 wt. % of cellulose or a derivative thereof, based on the total weight of the collagen-cellulose material; 1.0-6.0 wt. % of at least one acid selected from an inorganic acid, an organic acid, and mixtures thereof, based on the total weight of the collagen-cellulose material; and water.

In an exemplary aspect of the present invention the collagen-cellulose material comprising: 3.0-7.0 wt. % of a collagen, based on a total weight of the collagen-cellulose material; 0.8-2.0 wt. % of cellulose or a derivative thereof, based on the total weight of the collagen-cellulose material; 1.0-6.0 wt. % of at least one acid selected from an inorganic acid, an organic acid, and mixtures thereof, based on the total weight of the collagen-cellulose material; and a balance being water, wherein the total weight of the collagen, the cellulose or a derivative thereof, the at least one acid, and water is 100 wt. %.

The collagen-cellulose material may further comprise one or more compounds. Non-limiting examples of the one or more compounds include for example pectin, sodium citrate, ammonium sulfate, citric acid, a crosslinking agent (e.g., glutaraldehyde and/or hydroxyacetaldehyde), an aluminum salt, a sugar, a sugar derivative obtained from a caramelization process, a sugar mixture comprising a sugar or a derivative thereof and a crosslinking agent (e.g., MAILLOSE®), a browning agent, glycerin, an antimicrobial agent (e.g., sodium benzoate and/or hypochlorite), mineral oil, poly(lactic-co-glycolic acid) (PLGA), hydroxyapatite, a surfactant, a plasticizer, a dye, and mixtures thereof.

The one or more compounds are preferably selected from pectin, sodium citrate, ammonium sulfate, citric acid, a crosslinking agent (e.g., glutaraldehyde and/or hydroxyacetaldehyde), an aluminum salt, a sugar, a sugar derivative obtained from a caramelization process, a sugar mixture comprising a sugar or a derivative thereof and a crosslinking agent (e.g., MAILLOSE®), a browning agent, glycerin, sodium benzoate, and mixtures thereof.

The one or more compounds may be present in the collagen-cellulose material in an individual or combined amount of 0.1-25 wt. %, including for example 1.0-25.0 wt. %, 1.5-24.5 wt. %, 2.0-24.0 wt. %, 2.5-23.5 wt. %, 3.0-23.0 wt. %, 3.5-22.5 wt. %, 4.0-22.0 wt. %, 4.5-21.5 wt. %, 5.0-21.0 wt. %, 5.5-20.5 wt. %, 6.0-20.0 wt. %, 6.5-19.5 wt. %, 7.0-19.0 wt. %, 7.5-18.5 wt. %, 8.0-18.0 wt. %, 8.5-17.5 wt. %, 9.0-17.0 wt. %, 9.5-16.5 wt. %, 10.0-16.0 wt. %, 10.5-15.5 wt. %, 11.0-15.0 wt. %, 11.5-14.5 wt. %, 12.0-14.0 wt. %,12.5-13.5 wt. %, 13.0 wt. %, based on a total weight of the collagen-cellulose material.

Pectin may be present in an amount 0.1-5.0 wt. %, including for example 0.5-4.5 wt. %, 1.0-4.0 wt. %, 1.5-3.5 wt. %, 2.0-3.0 wt. %, or 2.5 wt. %, based on a total weight of the collagen-cellulose material.

The crosslinking agent (e.g., glutaraldehyde and/or hydroxyacetaldehyde) may be present in an amount of 50-500 ppm, including for example 100-450 ppm, 150-400 ppm, 200-350 ppm, or 250-300 ppm, based on a total weight of the collagen-cellulose material. Applicants have discovered that when pectin and/or an aluminum salt is combined with a crosslinking agent, crosslinking is enhanced and the hardness of the collagen hollow tubes and the collagen sheets more closely resembles that of blood vessels and tissue, respectively. Applicants have also discovered that when pectin and/or an aluminum salt is/are combined with a crosslinking agent and hydroxyapatite, crosslinking is enhanced and the hardness of the collagen solid more closely resembles that of bone.

The aluminum salt may be one or more aluminum salts selected from aluminum acetate, aluminum acetotartrate, aluminum acetylacetonate, aluminum bis(acetylsalicylate), aluminum borate, aluminum bromate, aluminum bromide, aluminum chlorate, aluminum chloride, aluminum citrate, aluminum fluoride, aluminum formate, aluminum iodide, aluminum lactate, aluminum maleate, aluminum nitrate, aluminum octoate, aluminum oleate, aluminum palmitate, aluminum phosphate, aluminum salicylate, aluminum stearate, aluminum sulfate, aluminum tartrate, aluminum tri(sec-butoxide), and mixtures thereof.

The sugar may be one or more sugars selected from a monosaccharide, a disaccharide, and mixtures thereof. Non-limiting examples of the monosaccharide include glucose, fructose, galactose, ribose, arabinose, xylose, lyxose, allose, altrose, mannose, gulose, iodose, talose, and mixtures thereof. Non-limiting examples of the disaccharide include sucrose, lactulose, lactose, maltose, cellobiose, kojibiose, nigerose, isomaltose, trehalose, α,α-trehalose, β,β-trehalose, α,β-trehalose, sophorose, laminaribiose, gentiobiose, turanose, maltulose, palatinose, gentiobiose, mannobiose, melibiose, rutinose, rutinulose, xylobiose, and mixtures thereof.

The sugar derivative obtained from a caramelization process may be present in a sugar mixture further comprising a crosslinking agent (e.g., hydroxyacetaldehyde, a.k.a., glycolaldehyde). MAILLOSE® is a particularly preferred sugar mixture and/or browning agent.

Applicants have discovered that the specific composition of the collagen-cellulose material can be adjusted (e.g., increasing or decreasing the concentration of one or more compounds within the collagen-cellulose material including, but not limited to, pectin, glutaraldehyde, aluminum, sugar, hydroxyapetite) in order to exhibit a desired tensile strength, modulus of elasticity, flexibility, porosity, compressibility, decompressibility (e.g., an ability to return to at least 80% of the original position), hardness, degree of crosslinking, wetness, sealability (e.g., an ability to maintain a hermetic seal following exposure to heat and/or pressure), and/or stability (e.g., a shelf life of at least six months with no change in physicochemical properties), as well as to mimic specific features of a desired anatomical structure.

The collagen-cellulose material may comprise less than 1.0 wt. % of calcium or a calcium compound (e.g., calcium oxide (a.k.a., lime), or calcium hydroxide (a.k.a., hydrated lime, caustic lime, slaked lime)), including for example less than 0.5 wt. %, less than 0.1 wt. %, less than 0.01 wt. %, and less than 0.001 wt. %, based on a total weight of the collagen-cellulose material. The collagen-cellulose material is particularly preferably decalcified and thus does not comprise calcium or a calcium compound.

According to one aspect of the present invention the collagen-cellulose material comprising: 3.0-7.0 wt. % of a collagen, based on a total weight of the collagen-cellulose material; 0.8-2.0 wt. % of cellulose or a derivative thereof, based on the total weight of the collagen-cellulose material; 1.0-6.0 wt. % of at least one acid selected from an inorganic acid, an organic acid, and mixtures thereof, based on the total weight of the collagen-cellulose material; 0.1-25 wt. % of one or more compounds selected from pectin, sodium citrate, ammonium sulfate, citric acid, a crosslinking agent (e.g., glutaraldehyde and/or hydroxyacetaldehyde), an aluminum salt, a sugar, a sugar derivative obtained from a caramelization process, a sugar mixture comprising a sugar or a derivative thereof and a crosslinking agent (e.g., MAILLOSE®), a browning agent, glycerin, an antimicrobial agent (e.g., sodium benzoate and/or hypochlorite), mineral oil, poly(lactic-co-glycolic acid) (PLGA), hydroxyapatite, a surfactant, a plasticizer, a dye, and mixtures thereof, based on the total weight of the collagen-cellulose material; and water.

According to an another aspect of the present invention the collagen-cellulose material comprising: 3.0-7.0 wt. % of a collagen, based on a total weight of the collagen-cellulose material; 0.8-2.0 wt. % of cellulose or a derivative thereof, based on the total weight of the collagen-cellulose material; 1.0-6.0 wt. % of at least one acid selected from an inorganic acid, an organic acid, and mixtures thereof, based on the total weight of the collagen-cellulose material; 0.1-25 wt. % of one or more compounds selected from pectin, sodium citrate, ammonium sulfate, citric acid, a crosslinking agent (e.g., glutaraldehyde and/or hydroxyacetaldehyde), an aluminum salt, a sugar, a sugar derivative obtained from a caramelization process, a sugar mixture comprising a sugar or a derivative thereof and a crosslinking agent (e.g., MAILLOSE®), a browning agent, glycerin, an antimicrobial agent (e.g., sodium benzoate and/or hypochlorite), mineral oil, poly(lactic-co-glycolic acid) (PLGA), hydroxyapatite, a surfactant, a plasticizer, a dye, and mixtures thereof, based on the total weight of the collagen-cellulose material; and a balance being water, wherein the total weight of the collagen, the cellulose or a derivative thereof, the at least one acid, the one or more compounds, and water is 100 wt. %.

The following description describes a process for producing the collagen-cellulose material, which may be in the form of a hollow tube, a sheet, or a solid. The hollow tubes, sheets, and solid of the collagen-cellulose material may be prepared by a variation of a procedure used for the fabrication of collagen casings.

The collagen may be extracted/isolated from any source (e.g., any animal sources) including for example human, equine, bovine, porcine, alligator, and/or fish. The collagen is preferably extracted from bovine, porcine, and/or fish due to reduced antigenicity or an absence of antigenicity when the collagen-cellulose material is introduced *in vivo.*

In an exemplary aspect, the collagen is extracted/isolated from the epidermis and/or the corium/dermis layer, which is the fibrous inner layer of the skin just beneath the epidermis and consists essentially of an enriched-collagen tissue, of an animal hide, preferably a bovine or porcine animal hide.

The extracted/isolated collagen may be subjected to a limed split process which involves exposing the extracted/isolated collagen to calcium or a calcium compound (e.g., calcium oxide (a.k.a., lime), or calcium hydroxide (a.k.a., hydrated lime, caustic lime, slaked lime)) to breakdown the collagen into a fibrous structure to obtain a collagen split.

The collagen split may be subjected to decalcification with washing to remove undesirable calcium or calcium compounds, which have been incorporated into the collagen split during the limed split process, to obtain a purified collagen tissue, composed primarily of collagen fibers and collagen fibrils. Extensive washing may be carried out to remove a higher concentration of undesirable calcium or calcium compounds to thereby obtain collagen tissue having a higher degree of purity.

The purified collagen tissue may be subjected to acidification with a buffer composition, which comprises citric acid and/or a salt thereof (e.g., sodium citrate), ammonium sulfate, and water, to obtain a collagen material at a pH of 3.0-6.0, including for example 3.5-5.5, 4.0-5.0, or 4.5, preferably 4.0-5.0, 4.1-4.9, 4.2-4.8, 4.3-4.7, 4.4-4.6, or 4.5, and most preferably 4.5.

The collagcn material may be cut and/or ground, optionally at a temperature of 10-25°C, including for example 11-24°C, 12-23°C, 13-22°C, 14-21°C, 15-20°C, 16-19°C, or 17-18°C, preferably 11-19°C, 14-18°C, 13-17°C, 12-16°C, or 13-15°C, and most preferably 13-17°C, 12-16°C, or 13-15°C, to obtain collagen which may be in the form of a powder, a fibril, and/or a fiber.

The collagen is mixed with: cellulose or a derivative thereof; at least one acid selected from an inorganic acid, an organic acid, and mixtures thereof; one or more optional compounds (e.g., a crosslinking agent (e.g., glutaraldehyde) and/or an aluminum salt); and water, for a time period of approximately 10-20 minutes, including for example 11-19 minutes, 12-18 minutes, 13-17 minutes, 14-16 minutes, or preferably 15 minutes, to form a fibrous slurry or a doughy mass having a pH of less than or equal to 3.0, including for example 1.0-3.0, 1.5-2.5, or 2.0.

Important steps in the formation of a fibrous slurry or a doughy mass having an appropriate consistency include the specific nature and concentrations of the components, the temperature, the pH, and/or the physical mixing/blending of the components.

The fibrous slurry or the doughy mass may be subjected to one or more treatments selected from cutting, grinding, homogenization, filtration (e.g., through a 0.008 mesh screen and/or a 0.006 mesh screen), dipping, soaking and/or extrusion (e.g., in the presence of an aluminum salt solution and/or a sugar mixture comprising a sugar or a derivative thereof and a crosslinking agent (e.g., MAILLOSE®)), washing (e.g., washing with an aqueous solution which may comprise an optional crosslinking agent, such as glutaraldehyde, for example), and/or drying.

The aluminum salt solution for use in the dipping, soaking and/or extrusion may comprise one or more of the aforementioned aluminum salts and an optional crosslinking agent (e.g., glutaraldehyde). A non-limiting example of the aluminum salt solution comprises 2.9-3.5 wt. % of an ammonium salt, 1.0-1.6 wt. % of ammonium sulfate, 0.4-1.0 wt. % of citric acid, 0.05-0.15 wt. % of ammonium hydroxide, and 225-275 ppm of glutaraldehyde. The fibrous slurry or the doughy mass may be soaked for a period of time (e.g., 30 minutes to 3.5 hours) in the aluminum salt solution prior to extrusion and/or be exposed to the aluminum salt solution during dipping and/or extrusion.

The sugar mixture comprising a sugar or a derivative thereof and a crosslinking agent (e.g., MAILLOSE®) may further comprise glycerin/glycerol and water. The fibrous slurry or the doughy mass may be soaked for a period of time (e.g., 30 minutes to 3.5 hours) in the sugar mixture prior to extrusion and/or be exposed to the sugar mixture during dipping and/or extrusion.

Following the treatment, the fibrous slurry or the doughy mass is subjected to aging in a storage tank at a temperature of less than 25°C for a period of time (e.g., at least 6, 7, 8, 9, 10, 11, or 12 hours) sufficient to obtain a collagen-cellulose gel which is maintained at a temperature of less than 25°C prior to being subjected to an extrusion process to obtain a collagen-cellulose material. The aluminum salt solution and/or the sugar mixture may be added to the storage tank at various concentrations in order to obtain a desired degree of crosslinking prior to being subjected to the extrusion process. In addition, or as an alternative, to adding the aluminum salt solution and/or the sugar mixture to the storage tank, the collagen-cellulose gel may be soaked for a period of time (e.g., 30 minutes to 3.5 hours) in the aluminum salt solution and/or the sugar mixture prior to extrusion and/or be exposed to the aluminum salt solution and/or the sugar mixture during extrusion.

The collagen-cellulose material obtained from the extrusion may be of any desired shape, length, width, thickness, inner diameter, and/or outer diameter, and may be in the form of a hollow tube, a sheet, or a solid depending on the specific type of extruder utilized during the extrusion process and the specific extrusion conditions (e.g., temperature, pressure, rate of extrusion, etc.). The collagen-cellulose material may be subjected to optional drying after the extrusion to obtain a desired moisture content and degree of wetness. The composition and manufacturing conditions of collagen-cellulose material is adjusted and designed/fabricated to exhibit a desired tensile strength, modulus of elasticity, flexibility, porosity, compressibility, decompressibility (e.g., an ability to return to at least 80% of the original position), hardness, degree of crosslinking, wetness, sealability (e.g., an ability to maintain a hermetic seal following exposure to heat and/or pressure), and/or stability (e.g., a shelf life of at least six months with no change in physicochemical properties), as well as to mimic specific features of a desired anatomical structure.

The following Table is a non-limiting comparison of various physicochemical properties of the collagen-cellulose material in the form of a hollow tube, a sheet, and a solid.

**Table**

| PROPERTY | TUBE | SHEET | SOLID |
|---|---|---|---|
| Tensile strength | Particularly preferred | Particularly preferred | Acceptable |
| Modulus of elasticity | Particularly preferred | Acceptable | Acceptable |
| Flexibility | Particularly preferred | Acceptable | Acceptable |
| Porosity | Particularly preferred | Acceptable | Acceptable |
| Compressibility | Particularly preferred | Particularly preferred | Acceptable |
| Decompressibility | Particularly preferred | Particularly preferred | Acceptable |
| Hardness | Acceptable | Particularly preferred | Acceptable |
| Degree of crosslinking | Preferred | Particularly preferred | Acceptable |
| Wetness | Acceptable | Acceptable | Acceptable |
| Sealability | Particularly preferred | Particularly preferred | Acceptable |
| Stability | Particularly preferred | Particularly preferred | Particularly preferred |

The collagen-cellulose material obtained from the process comprises: 1.0-9.0 wt. % of a collagen, based on a total weight of the collagen-cellulose material; 0.2-3.0 wt. % of cellulose or a derivative thereof, based on the total weight of the collagen-cellulose material; 0.5-6.5 wt. % of at least one acid selected from an inorganic acid, an organic acid, and mixtures thereof, based on the total weight of the collagen-cellulose material; and water.

The collagen-cellulose material obtained from the process may be kept wet in a solution comprising: 5-15 wt. %, preferably 10 wt. % of glycerin; and 0.01-1.0 wt. %, preferably 0.1 wt. % of sodium benzoate.

The extracted/isolated collagen, the collagen-cellulose gel, and the collagen-cellulose material which may be in the form of a hollow tube, a sheet, or a solid, may be characterized by gel electrophoresis, capillary electrophoresis, and/or high-performance liquid chromatography, which may be carried out in the presence of sodium dodecyl sulfate. The degree of cross-linking of the collagen-cellulose gel and the collagen-cellulose material can also be determined.

The following description describes an article or device for *in vitro* or *in vivo* use comprising the collagen-cellulose material for pharmaceutical, medicinal, therapeutic, phlebotomical, surgical, or orthopedic applications.

The article for *in vitro* or *in vivo* use may further comprise a drug (e.g., antibiotic, analgesic, antihistamine, stimulant, tranquilizer, narcotic, antidepressant, hormone, anticoagulant, laxative, diuretic, etc.) and/or a pharmaceutically acceptable excipient. The article may be in any form including for example a powder, a pill, a tablet, a capsule, a liquid, a solution, a suspension, a slurry, a drink, a syrup, a thin film or membrane, a bandage, a transdermal patch, a suppository, a cream, a gel, a liniment, a balm, a lotion, an ointment, and a salve.

The device (e.g., an orthopedic device) for *in vitro* or *in vivo* use comprises the collagen-cellulose material in a form selected from a prosthetic blood vessel (e.g., a prosthetic artery, a prosthetic vein, or a prosthetic capillary), a prosthetic bone (e.g., a prosthetic femur bone, a prosthetic tooth), and/or a prosthetic tissue including prosthetic connective tissue (e.g., prosthetic tendon, prosthetic ligament, or prosthetic organ), prosthetic epidermal tissue (e.g., prosthetic skin), prosthetic muscle tissue, and/or prosthetic nerve tissue.

The collagen-cellulose material which is in the form of a hollow tube may be useful as a guide, a stent protector, a nerve protector, and for various biochemical mechanisms.

## Claims

1. A method for testing phlebotomical, surgical or orthopedic instrumentation and/or practicing a phlebotomical, surgical or orthopedic procedure, wherein the testing of the phlebotomical, surgical or orthopedic instrumentation or the practicing of the phlebotomical, surgical or orthopedic procedure is performed on a medical device comprising:
a collagen-cellulose material comprising 1.0-9.0 wt. % of a collagen, based on a total weight of the collagen-cellulose material; 0.2-3.0 wt. % of cellulose or a derivative thereof, based on the total weight of the collagen-cellulose material; 0.5-6.5 wt. % of at least one acid selected from the group consisting of an inorganic acid, an organic acid, and mixtures thereof, based on the total weight of the collagen-cellulose material; and water; and wherein the collagen-cellulose material is in a form of an artificial blood vessel, an artificial tissue, and/or an artificial bone.

2. The method of claim 1, wherein the medical device further comprises at least one component selected from the group consisting of a synthetic polymer, a natural polymer, an elastomer, a plastic, a thermoplastic, a synthetic rubber, a natural rubber, a silicone rubber, a hydrogel, a ceramic, a metal, an adhesive, a cement, wood, a dye, a pigment, an actual human blood vessel, bone or tissue, an actual animal blood vessel, bone or tissue, and combinations thereof.

3. The method of claim 1, wherein the medical device further comprises a support member for supporting the artificial blood vessel, the artificial tissue and/or the artificial bone.

4. The method of claim 3, wherein the support member is in the form of a mannequin arm or an artificial arm for supporting the artificial blood vessel.

5. The method of claim 1, which is for practicing a phlebotomical procedure.

6. The method of claim 1, wherein the collagen-cellulose material comprises 3.0-7.0 wt. % of the collagen;
0.8-2.0 wt. % of the cellulose or the derivative thereof; and
1.0-6.0 wt. % of the at least one acid.

7. The method of claim 1, wherein the cellulose is present in the collagen-cellulose material is obtained from wood, and/or at least one pectin-rich plant material selected from the group consisting of a peel of a citrus fruit, apple pomace, sugar beet pulp, a sunflower head, a carrot, a potato, a tomato, and combinations thereof.

8. The method of claim 1, wherein the derivative of cellulose is present in the collagen-cellulose material and is at least one ether derivative of cellulose selected from the group consisting of methylcellulose, ethylcellulose, ethylmethylcellulose, hydroxyethylcellulose, hydroxyethylmethylcellulose, hydroxypropylcellulose, hydroxypropylmethylcellulose, ethylhydroxyethylcellulose, carboxymethylcellulose, carboxyethylcellulose, and mixtures thereof.

9. The method of claim 1, wherein the derivative of cellulose is present in the collagen-cellulose material and is at least one ester derivative of cellulose selected from the group consisting of cellulose acetate, cellulose triacetate, cellulose propionate, cellulose acetate propionate, cellulose acetate butyrate, cellulose nitrate, cellulose sulfate, and mixtures thereof.

10. The method of claim 1, wherein the acid is one or more inorganic acids selected from the group consisting of hydrochloric acid, nitric acid, phosphoric acid, sulfuric acid, boric acid, hydrofluoric acid, hydrobromic acid, perchloric acid, and mixtures thereof.

11. The method claim 1, wherein the acid is one or more organic acids selected from the group consisting of formic acid, acetic acid, propionic acid, butyric acid, valeric acid, caproic acid, and mixtures thereof.

12. The method of claim 1, wherein the acid is a mixture of one or more inorganic acids and one or more organic acids, wherein a weight ratio of the one or more inorganic acids to the one or more organic acids is 10/1 to 1/10.

13. The method of claim 1, wherein the acid is a mixture of hydrochloric acid and acetic acid in a weight ratio of 3/1 to 1/3.

14. The method of claim 1, wherein the collagen-cellulose material further comprises 0.1-25 wt. % of one or more compounds selected from the group consisting of pectin, sodium citrate, ammonium sulfate, citric acid, a crosslinking agent, an aluminum salt, a sugar, a sugar derivative obtained from a caramelization process, a sugar mixture, a browning agent, glycerin, an antimicrobial agent, hydroxyapatite, and mixtures thereof, based on the total weight of the collagen-cellulose material.

## Patentansprüche

1. Verfahren zum Testen einer phlebotomischen, chirurgischen oder orthopädischen Instrumentierung und/oder Ausführen eines phlebotomischen, chirurgischen oder orthopädischen Verfahrens, wobei das Testen der phlebotomischen, chirurgischen oder orthopädischen Instrumentierung oder das Ausführen des phlebotomischen, chirurgischen oder orthopädischen Verfahrens auf einer medizinischen Vorrichtung durchgeführt wird, umfassend:
ein Kollagen-Cellulosematerial, umfassend 1,0 - 9,0 Gew.-% eines Kollagens, basierend auf einem Gesamtgewicht des Kollagen-Cellulosematerials; 0,2 - 3,0 Gew.-% Cellulose oder ein Derivat davon, basierend auf dem Gesamtgewicht des Kollagen-Cellulosematerials ; 0,5 - 6,5 Gew.-% wenigstens einer Säure, die aus der Gruppe ausgewählt ist, bestehend aus einer anorganischen Säure und Mischungen davon, basierend auf dem Gesamtgewicht des Kollagen-Cellulosematerials; und Wasser; und wobei das Kollagen-Cellulosematerial in einer Form eines künstlichen Blutgefäßes, eines künstlichen Gewebes und/oder eines künstlichen Knochens vorliegt.

2. Verfahren gemäß Anspruch 1, wobei die medizinische Vorrichtung weiterhin wenigstens ein Bauteil umfasst, das aus der Gruppe ausgewählt ist, bestehend aus einem synthetischen Polymer, einem natürlichen Polymer, einem Elastomer, einem Plastik, einem Thermoplastik, einem synthetischen Gummi, einem natürlichen Gummi, einem Silicongummi, einem Hydrogel, einer Keramik, einem Metall, einem Haftstoff, einem Zement, Holz, einem Farbstoff, einem Pigment, einem echten menschlichen Blutgefäß, Knochen oder Gewebe, einem echten tierischen Blutgefäß, Knochen oder Gewebe und Kombinationen davon.

3. Verfahren gemäß Anspruch 1, wobei die medizinische Vorrichtung ein Trägerelement zum Stützen des künstlichen Blutgefäßes, des künstlichen Gewebes und/oder des künstlichen Knochens umfasst.

4. Verfahren gemäß Anspruch 3, wobei das Trägerelement die Form eines Puppenarms oder eines künstlichen Arms zum Stützen des künstlichen Blutgefäßes aufweist.

5. Verfahren gemäß Anspruch 1, das zum Ausführen eines phlebotomischen Verfahrens dient.

6. Verfahren gemäß Anspruch 1, wobei das Kollagen-Cellulosematerial umfasst
3,0 - 7,0 Gew.-% des Kollagens;
0,8 - 2,9 Gew.-% der Cellulose oder des Derivats davon; und
1,0 - 6,0 Gew.-% der wenigstens einen Säure.

7. Verfahren gemäß Anspruch 1, wobei die Cellulose, die in dem Kollagen-Cellulosematerial vorhanden ist, aus Holz und/oder wenigstens einem pektinreichen Pflanzenmaterial erhalten wird, das ausgewählt ist aus der Gruppe bestehend aus einer Schale einer Zitrusfrucht, Apfel-Fruchtfleisch, Zuckerrüben-Fruchtfleisch, einer Sonnenblumenblüte, einer Karotte, einer Kartoffel, einer Tomate und Kombinationen davon.

8. Verfahren gemäß Anspruch 1, wobei das Derivat der Cellulose in dem Kollagen-Cellulosematerial vorhanden ist und wenigstens ein Celluloseetherderivat ausgewählt ist aus der Gruppe bestehend aus Methylcellulose, Ethylcellulose, Ethylmethylcellulose, Hydroxyethylcellulose, Hydroxyethylmethylcellulose, Hydroxypropylcellulose, Hydroxypropylmethylcellulose, Ethyl hydroxyethylcellulose, Carboxymethylcellulose, Carboxyethylcellulose und Mischungen davon.

9. Verfahren gemäß Anspruch 1, wobei das Derivat der Cellulose in dem Kollagen-Cellulosematerial vorhanden ist und wenigstens ein Celluloseetherderivat ausgewählt ist aus der Gruppe bestehend aus Celluloseacetat, Cellulosetriacetat, Cellulosepropionat, Celluloseacetatpropionat, Celluloseacetatbutyrat, Cellulosenitrat, Cellulosesulfat und Mischungen davon.

10. Verfahren gemäß Anspruch 1, wobei die Säure eine oder mehrere organische Säure(n) ist, die ausgewählt ist / sind aus der Gruppe bestehend aus Chlorwasserstoffsäure, Salpetersäure, Phosphorsäure, Schwefelsäure, Borsäure, Fluorwasserstoffsäure, Bromwasserstoffsäure, Perchlorsäure und Mischungen davon.

11. Verfahren gemäß Anspruch 1, wobei die Säure eine oder mehrere organische Säure(n) ist, die ausgewählt ist / sind aus der Gruppe bestehend aus Ameisensäure, Essigsäure, Propansäure, Buttersäure, Pentansäure, Capronsäure und Mischungen davon.

12. Verfahren gemäß Anspruch 1, wobei die Säure eine Mischung aus einer oder mehreren anorganischen Säuren und einer oder mehreren organischen Säuren ist, wobei ein Gewichtsverhältnis der einen oder mehreren anorganischen Säuren zu der einen oder mehreren organischen Säuren 10/1 bis 1/10 beträgt.

13. Verfahren gemäß Anspruch 1, wobei die Säure eine Mischung aus Salzsäure und Essigsäure in einem Gewichtsverhältnis von 3/1 bis 1/3 ist.

14. Verfahren gemäß Anspruch 1, wobei das Kollagen-Cellulosematerial weiterhin 0,1 - 25 Gew.-% einer oder mehreren Zusammensetzungen umfasst, die ausgewählt sind aus der Gruppe, bestehend aus Pektin, Natriumcitrat, Ammoniumsulfat, Citronensäure, einem Vernetzungsmittel, einem Aluminiumsalz, einem Zucker, einem Zuckerderivat, das aus einem Karamellisierungprozess gewonnen wurde, einer Zuckermischung, einem Bräunungsmittel, Glycerin, einem antimikrobiellen Mittel, Hydroxylapatit und Mischungen davon, basierend auf dem Gesamtgewicht des Kollagen-Cellulosematerials.

## Revendications

1. Méthode pour tester des instrumentations phlébotomiques, chirurgicales ou orthopédiques et/ou pour pratiquer une procédure phlébotomique, chirurgicale ou orthopédique, les tests de l'instrumentation phlébotomique, chirurgicale ou orthopédique ou la pratique de la procédure phlébotomique, chirurgicale ou orthopédique étant réalisés sur un dispositif médical, comprenant :
un matériel en collagène cellulosique, comprenant 1,0 à 9,0 % en poids de collagène sur la base d'un poids total du matériel en collagène cellulosique ; 0,2 à 3,0 % en poids de cellulose ou de dérivés de celle-ci, sur la base d'un poids total du matériel en collagène cellulosique ; 0,5 à 6,5 % en poids d'au moins un acide choisi dans le groupe consistant en un acide inorganique, un acide organique et des mélanges de ces derniers, sur la base d'un poids total du matériel en collagène cellulosique ; et de l'eau ; et où le matériel en collagène cellulosique présente la forme d'un vaisseau sanguin artificiel, d'un tissu artificiel et/ou d'un os artificiel.

2. Méthode selon la revendications 1, dans laquelle le dispositif médical comprend en plus au moins un composant choisi dans le groupe consistant en un polymère synthétique, un polymère naturel, un élastomère, un plastique, un caoutchouc synthétique, un caoutchouc naturel, un caoutchouc en silicone, un hydrogel, une céramique, un métal, un adhésif, un ciment, du bois, un colorant, un pigment, un vaisseau sanguin, os ou tissu humain naturel, un vaisseau sanguin, os ou tissu d'animal naturel et des combinaisons de ces derniers.

3. Méthode selon la revendication 1, dans laquelle le dispositif médical comprend en plus un élément pour support le vaisseau sanguin artificiel, le tissu artificiel et/ou l'os artificiel.

4. Méthode selon la revendication 3, dans laquelle l'élément de support présente la forme du bras d'un mannequin ou d'un bras artificiel pour supporter le vaisseau sanguin artificiel.

5. Méthode selon la revendication 1 destinée à pratiquer une procédure phlébotomique.

6. Méthode selon la revendication 1, dans laquelle le matériel en collagène cellulosique comprend
3,0 à 7,0 % en poids de collagène ;
0,8 à 2,0 % en poids de cellulose ou de dérivé de cette dernière ; et
1,0 à 6,0 % en poids d'au moins un acide.

7. Méthode selon la revendication 1 où la cellulose est présente dans le matériel en collagène cellulosique qui est obtenu à partir de bois et/ou au moins un matériel végétal riche en pectine choisi dans le groupe consistant en l'écorce d'un agrume, de pulpe de pomme, de pulpe de betterave sucrière, une tête de tournesol, une carotte, une pomme de terre, une tomate et des combinaisons de ces derniers.

8. Méthode selon la revendication 1, dans laquelle le dérivé de cellulose est présent dans le matériel en collagène cellulosique et est au moins un dérivé d'éther de cellulose choisi dans le groupe consistant en méthylcellulose, éthylcellulose, éthylméthylcellulose, hydroxyéthylcellulose, hydroxyéthylméthylcellulose, hydroxypropylcellulose, hydroxypropylméthylcellulose, éthylhydroxyéthylcellulose, carboxyméthylcellulose, carboxyéthylcellulose et des mélanges de ces derniers.

9. Méthode selon la revendication 1, dans laquelle le dérivé de cellulose est présent dans le matériel en collagène cellulosique et est au moins un dérivé d'éther de cellulose choisi dans le groupe consistant en acétate de cellulose, triacétate de cellulose, proprionate de cellulose, proprionate d'acétate de cellulose, butyrate d'acétate de cellulose, nitrate de cellulose, sulfate de cellulose et des mélanges de ces derniers.

10. Méthode selon la revendication 1, dans laquelle l'acide est un ou plusieurs acides inorganiques du groupe consistant en acide chlorhydrique, acide nitrique, acide phosphorique, acide sulfurique, acide borique, acide fluorhydrique, acide hydrobromique, acide perchlorique et des mélanges de ces derniers.

11. Méthode selon la revendication 1, dans laquelle l'acide est un ou plusieurs acides organiques choisi dans le groupe consistant en acide formique, acide acétique, acide propionique, acide butyrique, acide valérique, acide caproïque et des mélanges de ces derniers.

12. Méthode selon la revendication 1, ou l'acide est un mélange d'un ou de plusieurs acides inorganiques et d'un ou plusieurs acides organiques, dans laquelle un rapport de poids d'un ou de plusieurs acides inorganiques et d'un ou de plusieurs acides organiques est de 10/1 ou de 1/10.

13. Méthode selon la revendication 1, dans laquelle l'acide est un mélange d'acide chlorhydrique et d'acide acétique d'un rapport de poids de 3/1 à 1/3.

14. Méthode selon la revendication 1, dans laquelle le matériel en collagène cellulosique comprends en plus 0,1 à 25 % en poids d'un ou de plusieurs composants choisis dans le groupe consistant en pectine, citrate de sodium, sulfate d'ammonium, acide citrique, un agent de réticulation, un sel d'ammonium, un sucre, un dérivé de sucre obtenu par un processus de caramélisation, un mélange de sucre, un agent de brunissage, de la glycérine, un agent antimicrobien, de l'hydroxyapatite et un mélange de ces derniers sur la base du poids total du matériel en collagène cellulosique.
